# EUROPEAN PATENT APPLICATION

(11) **EP 4 112 103 A1**
(43) Date of publication of application: **04.01.2023**
(21) Application number: 21182622.7
(22) Date of filing: 30.06.2021
(51) Int. Cl.: A61M 5/315

(54) **DRUG DELIVERY DEVICE WITH ACOUSTIC AND/OR VIBRATION SIGNAL**

(71) Applicant: Ypsomed AG, 3401 Burgdorf (CH)
(72) Inventor: Brügger, Martin, 3065 Bolligen (CH); Schrul, Christian, 3400 Burgdorf (CH); Scheurer, Simon, 3006 Bern (CH); Bosshard, Simon Martin, 3006 Bern (CH)
(74) Representative: Meier Obertüfer, Jürg

(57) **Abstract**

The present application relates to a drug delivery device comprises a housing and a plunger rod arranged within the housing. The plunger rod is adapted to advance a piston inside a reservoir containing a medicament in order to dispense the medicament. The drug delivery device comprises a drive including an electric motor to move the plunger rod either in rotation and/or linearly along a central axis of the housing in order to move the piston. Further, the drug delivery device comprises an electronic control element which controls the electric motor, the electronic control element being configured to actuate the electric motor such as to generate an acoustic and/or vibration signal, preferably without moving the plunger rod.

## Description

### Technical Field

The invention relates to a drug delivery device with an acoustic and/or vibration signal. The acoustic and/or vibration signal is generated by means of a drive of the drug delivery device.

### Background Art

A variety of diseases exist that require regular treatment by subcutaneous administration of a medicament, and a number of drug delivery devices have been developed to support a patient in accurately and controllably delivering an amount of drug in a self-administration process. Drug delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens.

An automatic drug delivery device has a motor or a drive spring for biasing a plunger rod and shifting a piston in a container barrel, wherein the drive spring may have to be charged or strained manually prior to injection of a dose. A manually powered drug delivery device requires a user to manually provide the energy to move the piston, for instance by applying a distal force component to the drug delivery device.

The medicament dose to be injected may typically be manually selected by turning a dosage knob and observing the actual dialed dose from a dose window or display of the drug delivery device. A dose is dispensed by inserting the needle into a suited portion of human skin and by moving the piston manually or by pressing a release button of an automatic drug delivery device.

There are several types of drug delivery devices known in the art. Disposable drug delivery devices are adapted to deliver medication from a container such as a pre-filled cartridge or syringe that is not intended to be replaced or refilled by the patient. Reusable, semi-disposable, or hybrid drug delivery devices have components of the device that may be replaced by the patient, or a cartridge that may be refilled, while some components of the device may be reused with the replaced or refilled drug container.

Several doses may be administered with a single drug delivery device or with a single cartridge of a drug delivery device. In order to offer guidance to a patient whether the drug delivery device or cartridge may still be used or if a replacement is necessary, a drug delivery device may comprise a counter which shows the amount of medicament or of doses which have already been administered with or which remain in the drug delivery device or cartridge.

Especially with automatic drug delivery devices, it is advantageous when the drug delivery device provides feedback signals to a patient using the drug delivery device. Some automatic drug delivery devices use screens or other optic element, such a light diode, to provide feedback to the patient. However, optic feedback elements may not always be in the line of sight of the patient or may even distract the patient during administration of the medicament. Hence, some drug delivery devices use acoustic or vibration signals as feedback. However, this usually entails the integration of further components, such as a sound generator or vibrator into the drug delivery device.

### Summary of the invention

It is the object of the invention to create a drug delivery device which includes an acoustic and/or vibration feedback which uses a low number of parts and which is cheap in manufacture.

The solution of the invention is specified by the features of claim 1. According to the invention, the drug delivery device comprises a housing and a plunger rod arranged within the housing. The plunger rod is adapted to advance a piston inside a reservoir containing a medicament in order to dispense the medicament. The drug delivery device comprises a drive including an electric motor to move the plunger rod either in rotation and/or linearly along a central axis of the housing in order to move the piston. Further, the drug delivery device comprises an electronic control element which controls the electric motor, the electronic control element being configured to actuate the electric motor such as to generate an acoustic and/or vibration signal, preferably without moving the plunger rod.

As the drug delivery device according to the present invention uses a single electric motor both for the delivery of the medicament as well as for the generation of the acoustic and/or vibration signal, no further element has to be added to the drug delivery device in order to provide an additional feedback means. In particular no loudspeaker has to be provided. Hence, the number of parts used for the drug delivery device may be kept low which facilitates the manufacture of the drug delivery device and keeps the costs low.

The drug delivery device preferably is an injection device. An injection device is removed from an injection site after each administration of a dose of a medicament. Alternatively, the drug delivery device may be an infusion device, i.e. a device with a cannula or needle which remains in the skin of a patient for a period of time, e.g. several hours or days in order to administer several doses of the medicament to the patient during the period of time. More preferably, however, the drug delivery device is an injection device, for example, a pen-shaped reusable or semi-disposalble injection device.

The housing preferably is elongated and has a round, oval, rectangular or polygonal cross-section. The cross section may vary in size and form along the central axis of the housing. Preferably, the housing is made of a polymer material, more preferably of a medical grade polymer material, i.e. polymer material which fulfil the requirement of ISO 10993 and/or of the EU regulation 2017/745. The housing has a distal end and a proximal end. The distal end is the end of the housing which points towards a cannula or needle used to administer the medicament to a patient and which is connected to the drug delivery device. In the case that the drug delivery device is an injection device, the distal end of the housing will thus point towards the skin of a patient during an injection event. The proximal end of the housing therefore is the end of the housing which faces away of the needle or cannula. In the case that the drug delivery device is an injection device, the proximal end of the housing faces away of the skin of the patient during an injection event.

The housing is preferably open at its proximal and distal end such as to allow the assembly of the drug delivery device. The proximal and distal ends are closed with appropriate lids or the like in order to protect the elements of the drug delivery device located within the housing.

The drug delivery device preferably comprises a reservoir with a medicament. The reservoir may be located within the housing in certain embodiments. In this case, the drug delivery device is preferably discarded after all of the medicament in the reservoir has been administered to the patient. Preferably, however, the reservoir is included in a cartridge which is removably connectable with the housing, preferably at the distal end of the housing.

The reservoir comprises a piston which is movable in order to dispense the medicament through a cannula or needle. The cannula or needle is preferably connected or connectable to the distal end of the housing, to a reservoir holder or to the reservoir.

The medicament preferably is a medicament used to treat diabetes. Preferably, the medicament is insulin or a derivative thereof.

The plunger rod is either moved in rotation or is moved linearly about the central axis by the drive. By means of movement of the plunger rod, the piston is moved within the reservoir in order to dispense the medicament. The drive comprises an electric motor to impart a rotational or linear movement to the plunger rod. Hence, the drug delivery device is an automatic drug delivery device where the medicament is dispensed upon actuation of a button or the like by the patient. The plunger rod is preferably arranged such that its length axis is congruent with the central axis of the housing.

The electric motor may be of any suitable type, such as e.g. a DC motor. Preferably, the electric motor comprises a control system in order to determine the angular position and acceleration of its rotor, such as e.g. a servomotor.

The drug delivery device comprises a dispensing button which may be depressed or actuated by a patient in order to dispense a dose of the medicament from the drug delivery device. Alternatively, the delivery device comprises an actuation mechanism which is actuated when a user presses the delivery device on the skin (push on skin trigger).

Preferably, the drug delivery device comprises a dose setting mechanism which allows a patient to select or to modify the dose of the medicament which is dispensed with the drug delivery device. The dose setting mechanism preferably includes a dose knob which allows to select the dose by manually turning the dose knob. When a dose of the medicament is dispensed, the electronic control element switches the electric motor on for a specific time which is calculated based on parameters of the reservoir, such as diameter and/or volume as well as rotational speed of the electric motor such as the selected dose is dispensed.

In order to provide power to the electric motor and to the electronic control element, the drug delivery device comprises at least one electric storage element, such as for example a battery or a supercapacitor. If at least one battery is used as electric storage element, the battery preferably is a secondary battery which may be recharged. However, alternatively, the battery may also be a primary battery. In the case that the at least one battery is a secondary battery, the drug delivery device further comprises means to recharge the at least one battery, for example a plug for charging cable as well as a charging controller.

The electronic control element preferably comprises a printed circuit board with a microcontroller or microprocessor. The latter is connected to the electric motor, e.g. by means of printed circuit tracks and/or electric cables in order to actuate the electric motor.

For the delivery of the medicament, the electric motor is actuated by the electronic control element in order to move the plunger rod for a defined period of time in order to deliver a pre-set dose of the medicament. When the acoustic and/or vibration signal is to be generated, the electronic control element actuates the electric motor differently, preferably such that the plunger rod is not or only insubstantially moved, i.e. such that no medicament is delivered. These two modes of operation may be applied alternatingly during a dose delivery, to generate a sequence of audible click-like tones.

Additionally or instead, the same motor windings may be repeatedly supplied with current such that the rotor of the motor does not rotate or move but a vibration occurs. The supplied current may have a pulsed-shaped form as the current is switched on and off.

The drug delivery device does not include a loudspeaker. Furthermore, the delivery device does not provide a dedicated mechanical click sound generator for a dispensing click or an end of content signal. Instead, the acoustic and/or vibration signal can be generated exclusively by the motor.

Alternatively, the acoustic and/or vibration signal is generated during a dispensing movement of the plunger rod. In this case the electronic control element controls the motor in a manner that at the same time results in a dispensing movement and generates an almost continuous audible signal, preferably by way of pulse-width modulation of a suitable motor drive signal.

It is to be noted that the housing of the drug delivery device may act as a resonance body for the acoustic signal, as the electric motor is received within the housing and attached therewith.

The acoustic and/or the vibration signal may be used to signal different states of the drug delivery device. For example, the acoustic and/or vibration signal may be used to indicate the start or end of the delivery of a dose of a medicament with the drug delivery device. Or the acoustic and/or vibration signal may be used to signal that the drug delivery device has been switched on, is ready to deliver the dose of medicament, the charge of a battery is low, etc. Additionally, if the drug delivery device comprises a dose setting mechanism, the acoustic and/or vibration signal may be used to signal the selected dose, for example with an acoustic signal with increasing frequency when the selected dose is increased.

Preferably, the drive further comprises a mechanical gear or drive components with at least two elements in meshing engagement, the electronic control element being configured such as to actuate the electric motor in order that a first element of the at least two elements is moved in a relative rotational motion with respect to a second of the at least two elements, within a play of the meshing engagement. To that end, the direction of rotation of the motor may be switched back and forth a number of times, specifically in a state where the second element is not rotating. During product dispense, i.e. with the second element being rotated, the motor may simply be stopped for a short time, such that the inertia of the second element causes the impact with the first element.

The gear couples the electric motor with the plunger rod. Preferably, the gear thereby reduces the rotation speed of the electric motor, preferably by a ratio of at least 50:1. This means that for every at least 50 full rotations of a drive shaft of the electric motor, an output wheel or shaft of the gear rotates only one full revolution. Most preferably, the gear has a gear ratio of at least 100:1.

The at least two elements preferably are gear wheels and/or pinions. Further, the gear may comprise or be a planetary gear and/or a cycloidal drive and the at least two elements hence components of the planetary gear and/or cycloidal drive. Due to manufacturing tolerances, elements within a gear which are in meshing engagement usually have some play, i.e. one element may slightly move relative to a further element without transmitting this movement to said further element. Additionally or instead, the play may be between the plunger rod and a drive member or/and between the drive member and the gear. This play is being used in the present embodiment to generate an acoustic and/or vibration signal. Preferably, the gear or drive components may be configured such as to comprise a defined play. It is to be noted that the two components do not need to be in direct meshing engagement with each other, but rather further elements may be arranged between the first element and the second element.

The gear preferably transforms the rotational movement of the electric motor into a linear movement of the plunger rod along the length axis of the housing.

Preferably, the electronic control element generates a pulse-width-modulation signal to control or drive the electric motor. Using pulse-width-modulation signals to control an electric motor has the advantage to reduce the average power delivered to the electric motor. Further it allows to operate the electric motor with different speeds with a microcontroller in an easy and reliable way. A further advantage is that the power loss is reduced to almost zero by the use of a pulse-width-modulation signal.

As a person having skill in the art knows, an electric signal is chopped up into discrete pulses in order to generate a pulse-width-modulation signal. This means that the power supply is switched on and off with a discrete frequency. The power delivered to a load thereby corresponds to the average current of the pulses of the pulse-width-modulation signal. This average current may be modified by varying the time that the power supply is switched on, hence also the name pulse-width-modulation.

The electric motor preferably is a stepper motor. Use of a stepper motor allows a precise control of the rotation of the motor without the need of an open-loop or closed-loop controller. The stepper motor may be a unipolar, bipolar or higher phase stepper motor.

Preferably, the electronic control element is configured to actuate the electric motor with a chopping frequency of the pulse-width-modulation signal of between 1 kHz and 20 kHz, preferably of between 2 kHz and 10 kHz in order to generate the acoustic signal.

For the delivery of the medicament, usually a chopper frequency above 20 kHz is used so that the electric motor runs quietly and smoothly. By using a chopper frequency from 1 kHz to 20 kHz, especially in the range between 2 kHz and 10 kHz the electric motor produces an audible acoustic signal.

As a person skilled in the art knows, the chopper frequency is the frequency at which the power is switched on and off by a controller, i.e. by the electronic control element in the present case, to generate the pulse signal used in pulse-width-modulation signaling.

Preferably, the electronic control element is configured to apply a defined sequence of pulse-width-modulation signals to a single electromagnet or to a pair of electromagnets of the stepper motor in order to generate the acoustic and/or vibration signal.

As the electromagnets in a stepper motor are usually actuated separately by means of a controller, i.e. the electronic control element in the present case, it is possible to generate specific actuation signals for each of the electromagnets of the stepper motor in order to generate the acoustic and/or vibration signals.

Hence it is possible to generate a very specific acoustic and/or vibration signal. Using different specific sequences of pulse-width-modulation signals to the same electromagnet(s) or to a different electromagnet(s) it is possible to generate different acoustic and/or vibration signals.

For example, by actuating electromagnets of a defined pair of electromagnets sequentially one after the other, a rotor of the electric motor may be brought into an oscillating movement between the two electromagnets belonging to said pair of electromagnets, thereby generating a vibration of the electric motor which propagates to the housing of the drug delivery device. A patient holding the drug delivery device will perceive this vibration signal.

The electronic control element is preferably configured to actuate the electric motor with pulse-width-modulation signals for a defined time period to move the piston rod, wherein the chopping frequency of the signal is varied during the defined time period in order to generate an acoustic and/or vibration signal during delivery of the medicament.

Hence it is possible to generate an acoustic and/or vibration signal while the medicament is being dispensed, for example to signal how much of the dose has already been dispensed, e.g. by generating an acoustic signal with increasing frequency every time that 10% of the dose has been dispensed.

The chopping frequency is thereby only changed for very short, specific periods of time to generate the acoustic and/or vibration signal, such that the delivery of the dose of medicament is not negatively affected by the signal generation.

Preferably, the chopping frequency is varied to be below 20 kHz, preferably between 1 kHz and 10 kHz at least for a part of the defined time in order to generate the acoustic and/or vibration signal.

Other advantageous embodiments and combinations of features come out from the detailed description below and the entirety of the claims.

### Brief description of the drawings

The drawings used to explain the embodiments show:
- Fig. 1: shows a perspective view of one embodiment of a drug delivery device according to the present invention;
- Fig. 2: a length section of the drug delivery device according to Fig. 1.

In the figures, the same components are given the same reference symbols.

### Preferred embodiments

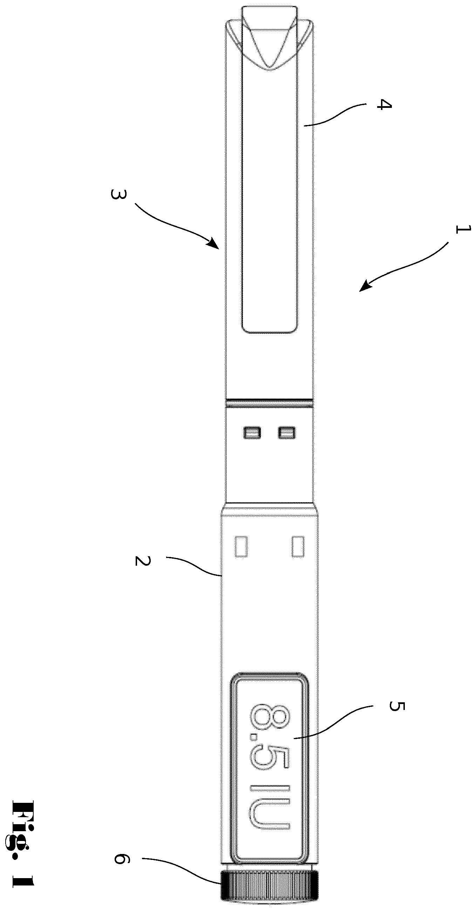

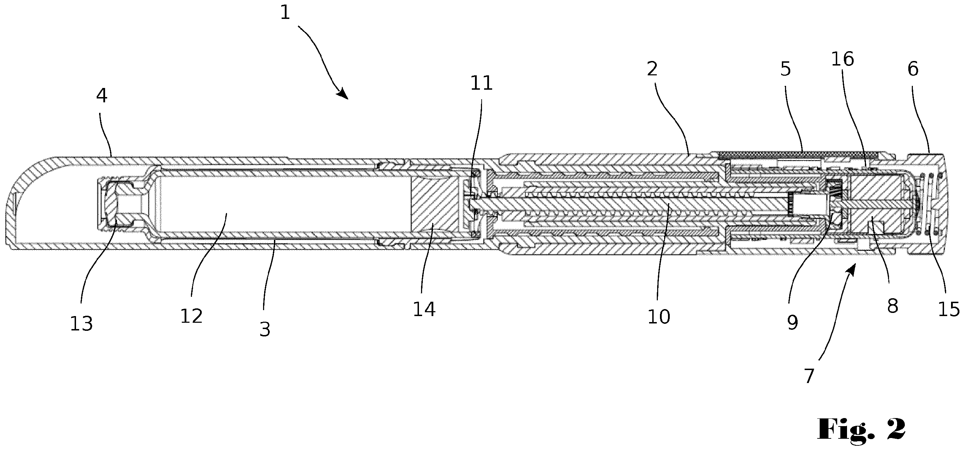

Fig. 1 shows a side view of one embodiment of a drug delivery device 1 according to the present invention. The drug delivery device 1 comprises a housing 2. At a distal end of the housing 2 a cartridge 3 is removably attached. The cartridge 3 contains a reservoir (see Fig. 2) with a medicament which may be dispensed with the drug delivery device 1. A cap 4 is removably arranged on the cartridge 3 in order to protect the medicament from contamination and to avoid any unintentional pricking of a person when a needle is connected to a distal end of the cartridge 3. The housing 2 comprises a screen 5, for example an LCD-screen to display information to a patient, e.g. the selected dose to be dispensed. Further, a combination of dose knob/dispensing button 6 is arranged on a proximal end of the housing 2. By turning the dose knob/dispensing button 6 around its axis a patient may select or change the dose of the medicament to be administered with the drug delivery device 1. The actual selected dose is displayed on screen 5.

Further, by depressing the dose knob/dispensing button 6 the dispensing of the selected dose of the medicament may be initiated.

Fig. 2 shows a length section of the drug delivery device 1 according to Fig. 1. A drive 7 is arranged within the housing 2 in a proximal area thereof. The drive 7 includes an electric motor 8 and a gear 9. Printed circuit boards of an electronic control element 16 are arranged between the drive 8 and an inner surface of the housing 2. The electronic control element 16 is connected with the dosage knob/dispensing button 6 and with the electric motor 8. The drive 7 moves a plunger rod 10 such that the plunger rod 10 is moved linearly along a length axis of the housing 2. It is to be noted that the plunger rod 10 may also additionally be moved in rotation by the drive 7. For example, the linear movement of the plunger rod 10 may be induced by means of the rotational movement of the plunger rod 10, for example when the plunger rod 10 has a thread on an outer surface which meshes with the housing 2 or a holder arranged within the housing 2. At its distal end, the plunger rod 10 includes a flange 11. The flange 11 abuts on a piston 14 which is arranged within a reservoir 12 with a volume filled with a medicament. By displacing the piston 14 in the distal direction, i.e. to the left on the figure the medicament within the reservoir may be dispensed. In order to inject the dispensed medicament into a patient, the reservoir 12 comprises a connection element 13 with which a needle or cannula may be removably connected to the reservoir 12.

The electronic control element 16 is configured such as to actuate the electric motor 8 of the drive 7 to generate an acoustic and/or vibration signal. This actuation is such that the plunger rod 10 is not move, i.e. such that no medicament is dispensed. In this case the electronic control element 16 provides an input current to two motor windings of the motor but does not shift the supplied current to other motor windings. Thus, the rotor of the electronic motor 8 does not rotate. In one embodiment, the electric motor 8 is actuated such that one element of the gear 9 moves within a play of the gear 9. In another embodiment, the electric motor 8 or - if the electric motor 8 is a stepper motor - one of the electromagnets within the electric motor 8 is actuated by means of pulse-width-modulation signals with a chopper frequency between 1 kHz and 20 kHz in order to generate an acoustic signal. In a further embodiment where the electric motor 8 is a stepper motor, a pair of electromagnets of the electric motor 8 may be actuated one after each other in sequence in order to move the rotor of the electric motor 8 between said two electromagnets in a repeated fashion, hence generating a vibration signal.

**List of reference symbols**

| | |
|---|---|
| 1 | Drug delivery device |
| 2 | Housing |
| 3 | Cartridge |
| 4 | Cover |
| 5 | Screen |
| 6 | Dose Knob / Dispensing button |
| 7 | Drive |
| 8 | Electric motor |
| 9 | Gear |
| 10 | Plunger rod |
| 11 | Flange |
| 12 | Reservoir |
| 13 | Connection element |
| 14 | Piston |
| 15 | Spring element |
| 16 | Electronic control element |

## Claims

1. Drug delivery device (1) comprising a housing (2) and a plunger rod (10) arranged within the housing (2), the plunger rod (10) being adapted to advance a piston (14) inside a reservoir (12) containing a medicament in order to dispense the medicament, the drug delivery device (1) comprising a drive including an electric motor (8) to move the plunger rod (10) either in rotation and/or linearly along a central axis of the housing (2) in order to move the piston, as well as an electronic control element (16) which controls the electric motor (8), **characterized in that** the electronic control element (16) is configured to actuate the electric motor (8) such as to generate an acoustic and/or vibration signal, preferably without moving the plunger rod (10).

2. Drug delivery device (1) according to claim 1, **characterized in that** the drive further comprises a gear (9) or drive components with at least two elements in meshing engagement, the electronic control element (16) being configured such as to actuate the electric motor (8) in order that a first element of the at least two elements is moved in a reciprocal rotational motion within a play of the meshing engagement with a second of the at least two elements.

3. Drug delivery device (1) according to claim 1 or 2, **characterized in that** the electronic control element (16) generates a pulse-width-modulation signal to actuate the electric motor (8).

4. Drug delivery device (1) according to any of the claims 1 to 3, **characterised in that** the electric motor (8) is a stepper motor.

5. Drug delivery device (1) according to claim 3 and 4, **characterized in that** the electronic control element (16) is configured to actuate the electric motor (8) with a chopping frequency of the pulse-width-modulation signal of between 1 kHz and 20 kHz, preferably of between 2 kHz and 10 kHz in order to generate the acoustic signal.

6. Drug delivery device (1) according to any of claims 3 to 5, **characterized in that** the electronic control element (16) is configured to apply a defined sequence of pulse-width-modulation signals to a single electromagnet or to a pair of electromagnets of the stepper motor (8) in order to generate the acoustic and/or vibration signal.

7. Drug delivery device (1) according to any of claims 3 to 6, **characterized in that** the electronic control element (16) is configured to actuate the electric motor (8) with pulse-width-modulation signals for a defined time period to move the piston rod, wherein the chopping frequency of the signal is varied during the defined time period in order to generate an acoustic and/or vibration signal during delivery of the medicament.

8. Drug delivery device (1) according to claim 7, **characterized in that** the chopping frequency is varied to be below 20 kHz, preferably between 1 kHz and 10 kHz at least for a part of the defined time.
